(19) Europäisches Patentamt
European Patent Office
Office européen des brevets

(11) Publication number: **0 273 216**
**A2**

(12) # EUROPEAN PATENT APPLICATION

(21) Application number: 87117704.4

(51) Int. Cl.[4] C07C 177/00 , A61K 31/557

(22) Date of filing: 30.11.87

(30) Priority: 04.12.86 JP 287732/86
13.08.87 JP 200830/87

(43) Date of publication of application:
06.07.88 Bulletin 88/27

(84) Designated Contracting States:
AT BE CH DE ES FR GB GR IT LI LU NL SE

(71) Applicant: SAGAMI CHEMICAL RESEARCH CENTER
4-5, Marunouchi 1-chome
Chiyoda-ku Tokyo 100(JP)

Applicant: TOA EIYO LTD.
1-2, Kyobashi 3-chome
Chuo-ku Tokyo(JP)

(72) Inventor: Shibasaki, Masakatsu
277-53, Higashi 1-chome Kita 12-jo
Higashi-ku
Sapporo-shi Hokkaido(JP)
Inventor: Takahashi, Atsuo
3-1, Nishi 9-chome Kita 35-jo Higashi-ku
Sapporo-shi Hokkaido(JP)
Inventor: Aoki, Tuyoshi
3-7-11, Higashi-rinkan
Sagamihara-shi Kanagawa-ken(JP)
Inventor: Mashiko, Toshihisa
14-11, Hirano Aa-gotomaki Iisaka-machi
Fukushima-shi Fukushima-ken(JP)
Inventor: Kogi, Kentaro
9-6, Koriyama Aza-guji
Shiroishi-shi Miyagi-ken(JP)
Inventor: Yamaguchi, Takashi TOA EIYO LTD.
Fukushima Kenkyusho 1, Yuno Aza-tanaka
Iisaka-machi Fukushima-shi
Kukushima-ken(JP)
Inventor: Nara, Takeshi TOA EIYO LTD.
Fukushima Kenkyusho 1, Yuno Aza-tanaka
Iisaka-machi Fukushima-shi
Fukushima-ken(JP)
Inventor: Narita, Senichi TOA EIYO LTD.
Fukushima Kenkyusho 1, Yuno Aza-tanaka
Iisaka-machi Fukushima-shi
Fukushima-ken(JP)

(74) Representative: Wächtershäuser, Günter, Dr.
Tal 29
D-8000 München 2(DE)

(54) **Prostacyclin analogue and anti-ulcer composition containing it as active ingredient.**

EP 0 273 216 A2

(57) A prostacyclin analogue having the formula:

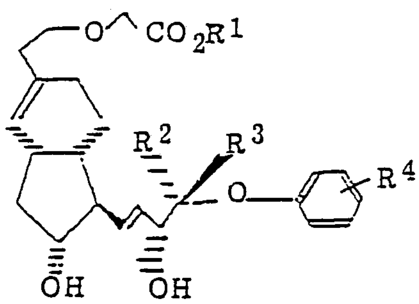

( I )

wherein R' is a hydrogen atom or a lower alkyl group; $R^2$ and $R^3$ each is a hydrogen atom or a lower alkyl group; $R^4$ is a hydrogen atom, a halogen atom or a lower alkoxy group.

# PROSTACYCLIN ANALOGUE AND ANTI-ULCER COMPOSITION CONTAINING IT AS ACTIVE INGREDIENT

The present invention relates to a novel stable prostacyclin analogue, an anti-ulcer composition containing it as an active ingredient, which is useful for the prevention or treatment of ulcer in the digestive tract.

Prostacyclin (hereinafter referred to simply as $PGI_2$) of the formula II and prostagrandin(s) $E_2$ (hereinafter referred to simply as $PGE_2$) of the formula III are known to be main prostagrandin(s) which exsist in gastric mucosal and have gastric mucosal protecting activities and gastric mucosal blood flow increasing activities ['83 Inflammation Seminar "Prostaglandin", preparatory text, page 50 (organized by Japan Inflammation Academy)]. Such prostagrandins are expected to be applicable for the prevention and treatment of a digestive tract ulcer, such as gastric ulcer.

However, $PGI_2$ and $PGE_2$ are very unstable substances. This is a major problem in their development as a drug.

In order to solve such a problem, some researches have been made on stable analogues having an anti-ulcer activity.

Stable analogues of $PGE_2$ are represented by Trimoprostil of the formula IV (US 4310543 A), Arbaprostil of the formula V (US 4301175 A) and Enprostil of the formula VI (EP 186181 A2)

On the other hand, Nileprost of the formula VII (Germany Offen DE 3427797 Al) is a stable analogue of $PGI_2$

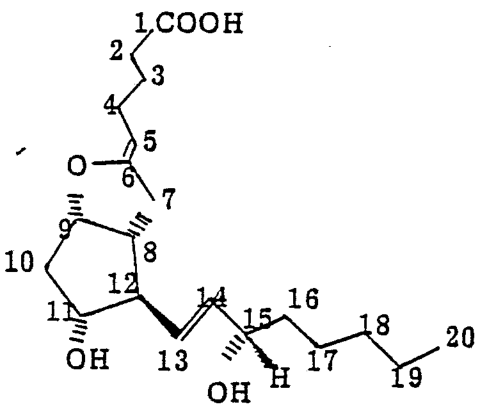

(II)

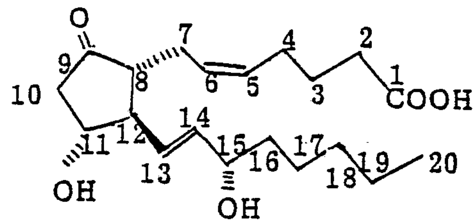

(III)

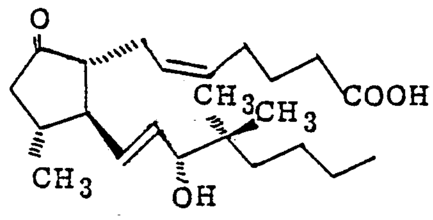

(IV)

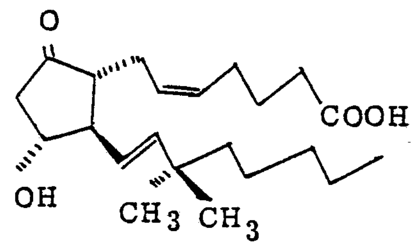

(V)

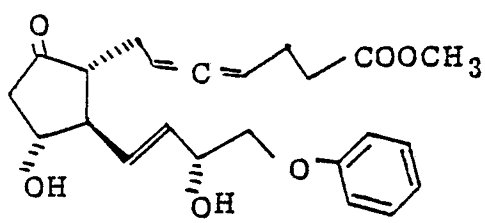

(VI)

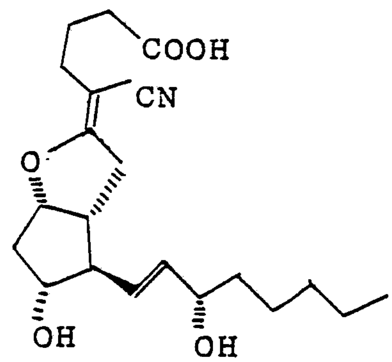

(VII)

The present inventors have conducted a wide range of researches with an aim to develop prostacyclins which are stable without any substantial decomposition at room temperature and having excellent pharmacological properties. As a result, novel prostacyclins have been prepared and it has been found that they have strong and long lasting anti-ulcer activities, and no substantial toxicity. The present invention has been accomplished on the basis of these discoveries.

The present invention provides a prostacyclin analogue having the formula:

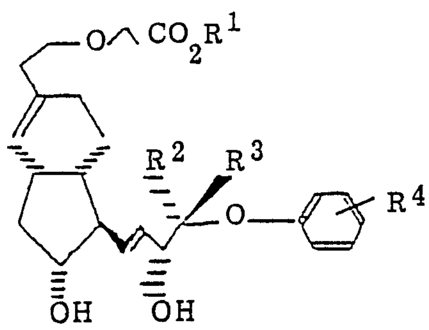

(I)

wherein $R^1$ is a hydrogen atom or a lower alkyl group; each of $R^2$ and $R^3$ is a hydrogen atom or a lower alkyl group; $R^4$ is a hydrogen atom, a halogen atom or a lower alkoxy group.

The present invention also provides an anti-ulcer composition comprising an effective amount of the prostacyclin analogue of the formula I and a pharmaceutically acceptable carrier.

The present inventors have made detailed studies on the pharmacological activities of the prostacyclin analogues, and as a result, have found that the group of compounds represented by the formula I have remarkable and long lasting anti-ulcer activities, even by oral administration, against ethanol ulcer, hydrochloric acid ulcer and indomethacin ulcer in animals. On the basis of this discovery, a further research has been made for the application to the prevention and treatment of digestive organ ulcers, and have accomplished the present invention.

The prostacyclin analogues of the present invention have hypotensive activities which are weak as compared with the hypotensive activities of other conventional prostacyclin analogues, and they do not cause diarrhea, which has been a stumbling block to the clinical application of $PGE_2$ analogues. Thus, they are superior in the selectivity of the activities, whereby the difficulties of the known PG analogues have been overcome. Thus, it is possible to provide a drug which is highly useful against gastric mucosal troubles i.e. acute mucosal troubles due to a food, an alcohol or a drug, and localized chronic tissue failures such as digestive ulcer.

Now, the synthesis of prostacyclin analogues having the formula I, will be described.

In the present invention, $R^5$ in the following formulas is a lower alkyl group such as methyl, ethyl, propyl or butyl: $R^6$ is a protective group for a hydroxyl group. As such a protective group, a t-butyldimethylsilyl group, a triethylsilyl group, a tribenzylsilyl group and a diphenyl-t-butylsilyl group may be mentioned.

$R^7$ is a hydrogen atom, an acyl group having from 1 to 7 carbon atoms, a tri-$C_1$-$C_7$ hydrocarbon-silyl group or a group capable of forming an acetal bond together with the oxygen atom of a hydroxyl group.

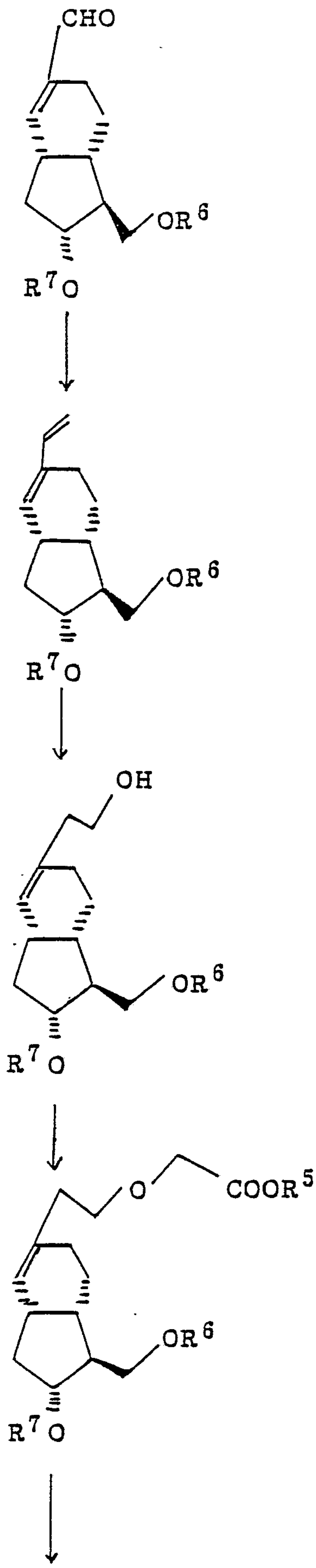

(VIII)

Step 1

(IX)

Step 2

(X)

Step 3

(XI)

Step 4

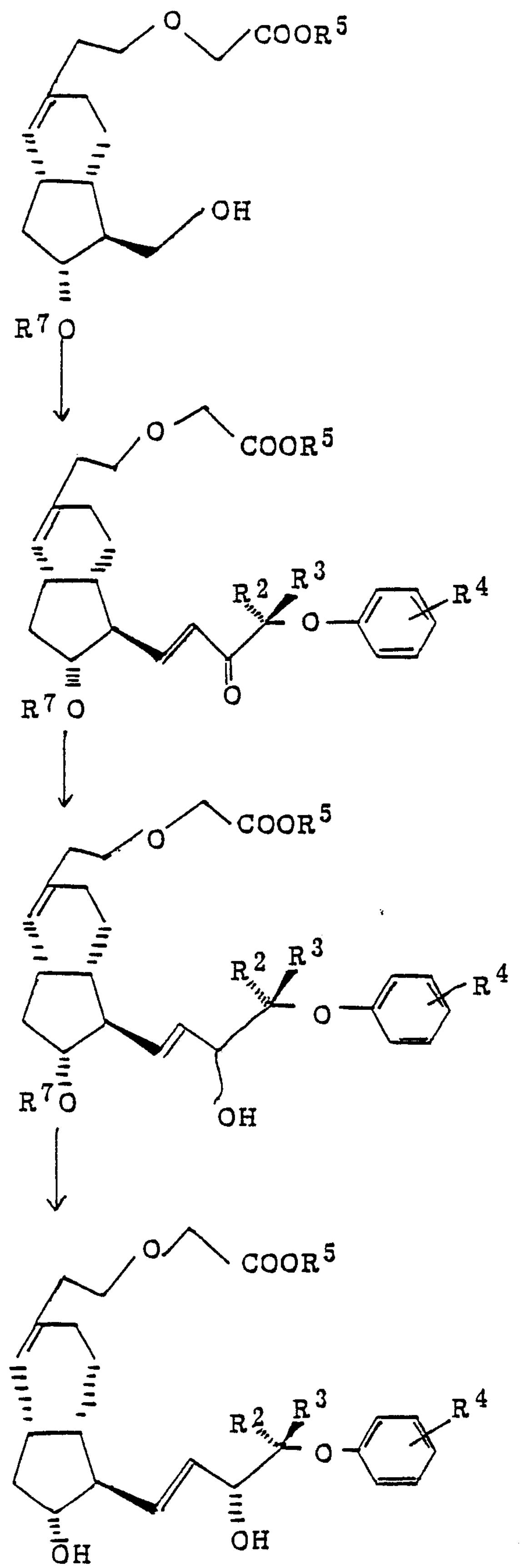

(XII)

Step 5

(XIII)

Step 6

(XIV)

Step 7

(I)

Step I

In this step, the 6-membered ring enal of the formula VIII (refer to the I06th annual seminar of the Pharmaceutical Society of Japan, preporatory text, p 38) is methylene-modified to obtain a conjugated diene of the formula IX. The methylene-modification reaction can be conducted in an ether-type solvent such as tetrahydrofuran by using an ylide compound prepared from methyltriphenylphosphonium bromide and a base such as a potassium salt of t-BuOH. The reaction temperature may be selected within a range of from 0 to 50°C.

Step 2

In this step, the conjugated diene of the formula IX obtained in the preceding Step I, is selectively hydroborate-modified, and then subjected to an oxidation reaction to obtain a primary alcohol of the formula X. For this hydroborate-modification, a hydroborate modification agent such as disiamyl borane or 9-BBN is used. The reaction is preferably conducted in a solvent. For this purpose, an ether-type solvent such as tetrahydrofuran, diglyme or diethyl ether may be employed. For the oxidation after the hydroborate-modification, an oxidizing agent such as hydrogen peroxide may be employed under a basic condition.

Step 3

In this step, β-oxa ester of the formula XI is prepared from the hydroxyethyl compound of the formula X. The reaction is such that t-butyl bromoacetate and the compound of the formula X are condensed in the presence of sodium hydroxide and an interphase transfer catalyst. As the interphase transfer catalyst, tetrabutylammoniumhydrogen sulfate or the like may be employed. The reaction temperature may be selected within a range of from 0 to 80°C. As the solvent for reaction, a two phase system of water-methylene chloride is employed.

Step 4

In this step, the protective group of the primary hydroxyl group of the β-oxa ester of the formula XI obtained in the preceding Step 3, is removed to obtain a bicyclo[4,3,0]nonene derivative of the formula XII. The reaction condition for the removal of the protective group varies depending upon the type of $R^6$. In the case where $R^6$ is a trialkylsilyl group such as t-butyl-dimethylsilyl and $R^7$ is an acetal-type protective group such as tetrahydropyranyl, the reaction is conducted in tetrahydrofuran by using tetra-n-butylammonium fluoride.

Step 5

In this step, the ω-chain primary alcohol of the formula XII is oxidized and then subjected to a Wittig reaction to obtain a conjugated enone of the formula XIII. The oxidation reaction can be conducted in a halogenated hydrocarbon such as methylene chloride by using a Collins' reagent or a sulfur trioxide-pyridine complex. The reaction temperature may be selected within a range of from -78 to 50°C.

In this step, the product obtained by the oxidation is then subjected to the Wittig reaction without isolating it. As the reactant, dimethyl[(2-oxo-3-methyl-3-phenoxy)butyl] phosphonate or dimethyl[(2-oxo-3-phenoxy)butyl] phosphonate may be employed. This Wittig reaction is preferably conducted in the presence of a base in order to obtain the desired product in good yield. A base such as sodium hydride, butyllithium or t-butoxypotassium may be employed. The reaction is preferably conducted in a solvent. As a solvent, an ether-type solvent such as tetrahydrofuran, dimethoxyethane or diethyl ether or an aromatic solvent such as benzene, toluene or xylene, may be employed. The reaction temperature may be selected within a range of from -25 to 50°C.

Step 6

In this step, the compound of the formula XIII obtained in the preceding Step 5 is reduced to obtain an allyl alcohol of the formula XIV. For this reduction, a reducing agent such as sodium borohydride or zinc borohydride may be employed. The solvent for the reaction may be methanol, ethanol, dimethoxyethane, ethyl ether, tetrahydrofuran, or the like. The reaction temperature may be selected within a range of from -50 to 50°C.

Step 7

In this step, the allyl alcohol of the formula XIV obtained in the preceding Step 6 is subjected to the removal of the protective group for the hydroxyl group and then, if desired, the hydrolysis of the ester to obtain the novel stable prostacyclin of the formula I. The removal of the protective group for the hydroxyl group varies depending upon the type of $R^7$. In the case where $R^7$ is a tetrahydropyranyl group, the reaction can be conducted in an acetic acid-$H_2O$-THF system by heating at a temperature of from 20 to 80°C. The hydrolysis of the ester can be conducted under a usual reaction condition i.e. in a $H_2O$-MeOH system or in a $H_2O$-EtOH system by using soduim hydroxide or potassium hydroxide. The reaction temperature may be selected within a range of from 0 to 50°C.

Specific examples of the prostacyclin analogues of the present invention, includes:

a) 3-(3-Oxa-4-carboxybutyl)-7-exo-(3α-hydroxy-4-methyl-4-phenoxy-trans-l-pentenyl)-8-endo-hydroxy-cis-bicyclo[4.3.0]nona-2-ene

b) 3-(3-Oxa-4-methoxycarbonylbutyl)-7-exo-($3_\alpha$-hydroxy-4-methyl-4-phenoxy-trans-l-pentenyl)-8-endo-hydroxy-cis-bicyclo[4.3.0]nona-2-ene

c) 3-(3-Oxa-4-carboxybutyl)-7-exo-($3_\alpha$-hydroxy-4-phenoxytrans-l-butenyl)-8-endo-hydroxy-cis-bicyclo-[4.3.0]-nona-2-ene

d) 3-(3-Oxa-4-carboxybutyl)-7-exo-(3α-hydroxy-4-phenoxytrans-l-pentenyl)-8-endo-hydroxy-cis-bicyclo-[4.3.0]-nona-2-ene

e) 3-(3-Oxa-4-carboxybutyl)-7-exo-[3α-hydroxy-4-(4-methoxyphenoxy)-trans-l-butenyl]-8-endo-hydroxy-cis-bicyclo[4.3.0]nona-2-ene

f) 3-(3-Oxa-4-carboxybutyl)-7-exo-[3α-hydroxy-4-(4-fluorophenoxy)-trans-l-butenyl]-8-endo-hydroxy-ci s-bicyclo[4.3.0]nona-2-ene

g) 3-(3-Oxa-4-carboxybutyl)-7-exo-[3α-hydroxy-4-(3-fluorophenoxy)-trans-l-butenyl]-8-endo-hydroxy-ci s-bicyclo[4.3.0]nona-2-ene

The prostacyclin analogues of the present invention, such as compounds I-a, I-a-e, I-c, I-d and I-f, have anti-ulcer activities which are comparable or superior to typical PG having anti-ulcer activities, such as $PGE_2$. Further, from the examination of the dose-response-time effect on ethanol-induced gastric lesions, the prostacyclin analogues of the present invention show a long lasting activities compared with that of $PGE_2$. While the $PGE_2$ analogues are likely to induce diarrhea, the prostacyclin analogues of the present invention do not have such diarrhea-inducing activities. While prostacyclin has remarkable hypotensive activities, the hypotensive activities of the prostacyclin analogues of the present invention are very weak, and thus they are superior in the selectivities of activities, whereby the object of the present invention as an anti-ulcer composition can be sufficiently accomplished.

These compounds of the present invention can be administered orally or non-orally for the treatment.

The drugs for oral administration may be solid formulations such as powders, granules, capsules, tablets or liquid formulations such as syrups or elixirs. The drug for non-oral administration may be injection drugs, rectal administration drugs, skin application drugs or inhalation drugs. These drugs can be prepared in accordance with a conventional method by mixing such an active ingredient with a pharmaceutically acceptable carrier or adjuvant. Further, it may be made in the form of a long-lasting agent in accordance with a known technique.

For the preparation of solid formulations for oral administration, the active ingredient may be mixed with a carrier such as lactose, starch, crystalline cellulose, calcium lactate, magnesium metasilicate aluminate or silicic anhydride to obtain a powder, or if necessary, a binder such as sucrose, hydroxypropyl cellulose or polyvinylpyrrolidone and a disintegrator such as carboxymethyl cellulose or calcium carboxymethyl cellulose, may be added and the mixture was subjected to wet or dry granulation to obtain granules. For the preparation of tablets, these powders or granules may be tabletted by themselves or after an addition of a lubricant such as magnesium stearate or talc. These granules or tablets may be formed into enteric coating formulations by coating them with an enteric coating agent such as hydroxypropylmethyl cellulose stearate,

methacrylic acid or a methyl methacrylate copolymer, or may be formulated into long-lasting formulations by coating them with ethyl cellulose, carnauba wax, hardened oil, etc.

For the preparation of capsules, the powders or granules, may be filled in hard capsules, or active ingredients may be dissolved in glycerol, polyethylene glycol, sesame oil, olive oil or the like, and then coated with a gelatin film to obtain soft capsules.

For the preparation of liquid formulations for oral administration, an active ingredient and a sweetening agent such as sorbitol or glycerol, may be dissolved in water to obtain a clear syrup, or refined oil or ethanol may be added thereto to obtain an elixir, or gum arabic or tragant, polysolvate 80 or sodium carboxy methyl cellulose, may be added thereto to obtain an emulsion or suspension. If desired, additives such as corrigents, coloring agents or storage agents may be added to these liquid formulations.

For the preparation of an injection solution, an active component may be dissolved in distilled water for injection, if necessary, together with a pH controlling agent such as hydrochloric acid, sodium hydroxide, lactic acid, sodium lactate, sodium hydrogen phosphate or sodium dihydrogen phosphate, and an isotonic agent such as sodium chloride or glucose, then septically filtered and filled in an ample, or mannitol, dextrin, cyclodextrin, gelatin or the like may be added thereto, followed by freeze drying under vacuum, to obtain an injection drug of the type which is to be dissolved at the time of the injection. Further, it is possible to prepare an emulsion for injection by emulsifying an active ingredient in water by an addition of lecitin, polysolvate 80 or polyoxyethylene hardened caster oil.

For the preparation of a rectal administration drug, an active ingredient and a suppository material such as cacao butter, fatty acid tri-, di-or mono-glyceride or polyethylene glycol, may be moistened, melted, poured in a mold and cooled, or an active ingredient may be dissolved in polyethylene glycol, soybean oil or the like, followed by coating with a gelatin film.

For the preparation of a skin application drug, an active ingredient may be added to white vaseline, beeswax, liquid paraffin, polyethylene glycol or the like, and the mixture is kneaded, if necessary under heating, to obtain an ointment, or it may be kneaded with an adhesive agent such as rosin or an alkyl acrylate polymer, and the mixture is then spread on a non-woven fabric of polyethylene, etc. to obtain a tape drug.

For the preparation of an inhalation drug, an active ingredient is dissolved or dispersed in a propellant such as Freon gas and filled in a pressure resistant container to obtain an aerosol.

The dose of the prostacyclin analogue of the present invention varies depending upon the age, the weight and the diseased state of the patient, but is usually within a range of from l µg to 500 mg per day, and may be administered at once or in several times.

Now, the present invention will be described in further detailed with reference to Reference Examples, Synthesis Examples, Test Examples and Formulation Examples. However, it should be understood that the present invention is by no means restricted to these specific Examples.

Reference Example 1

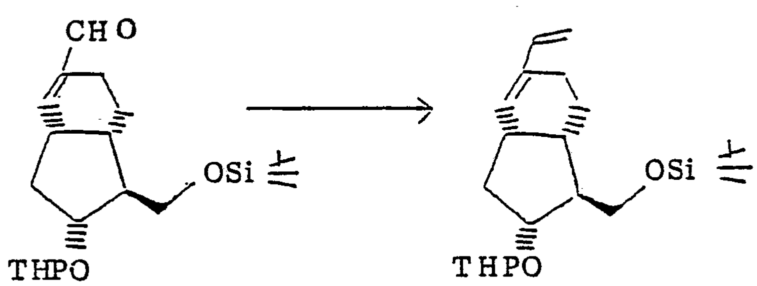

Under an argon atmosphere, methyltriphenylphosphonium bromide (449 mg, l.l4 mmol) preliminarily adequately dried at l00°C under reduced pressure, was suspended in THF (5 ml). Then, a THF solution (2 ml) of potassium t-butoxide (l40 mg, l.l4 mmol) was added thereto at room temperature. The mixture was stirred for l0 minutes, and then a THF solution (2 ml) of 3-formyl-7-exo-t-butyl-dimethylsilyloxymethyl-8-endo-tetrahydropyranyloxy-cis-bicyclo[4,3,0]nona-2-ene (l50 mg, 0.38 mmol) was added. The mixture was stirred at room temperature for 30 minutes. After an addition of a saturated ammonium chloride aqueous solution, the mixture was extracted with ethyl ether. The ethyl ether layer was washed with a saturated sodium chloride aqueous solution, and then dried over anhydrous magnesium sulfate. Then, the solvent was

distilled off. The residue was purified by silica gel column chromatography (ethyl ether:n-hexane = 1:15) to obtain 3-ethenyl-7-exo-t-butyldimethylsilyloxymethyl-8-endo-tetrahydropyranyloxy-cis-bicyclo[4.3.0]nona-2-ene (139 mg, 93%).

IR ν max (neat) : 2950, 2875, 1640, 1605, 1475, 1260, 1200, 840, 780 cm⁻¹

NMR ($CDCl_3$) δ : 6.33(dd, J = 20, 11.6Hz, 1H), 5.66(bs, 1H), 5.03(d, J = 20Hz, 1H), 4.88(d, J = 11.6Hz, 1H), 4.60-(bs, 1H) 0.90(s, 9H), 0.05(s, 6H).

Mass (m z) (%) : 374 (M -$H_2O$, trace), 308 (7), 251 (18), 205 (3), 159 (77), 85 (100), 57 (19).

Reference Example 2

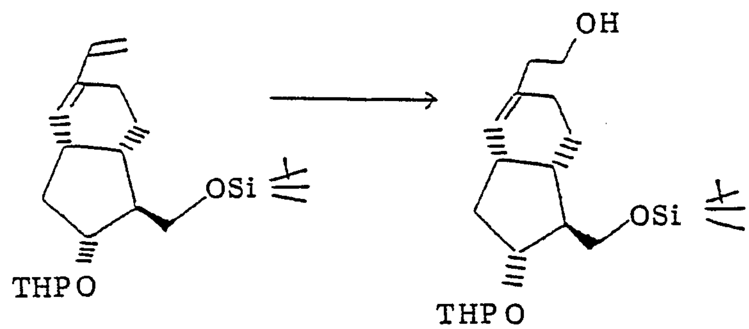

Under an argon atmosphere, 3-ethenyl-7-exo-t-butyldimethylsilyloxymethyl-8-endo-tetrahydropyranyloxy-cis-bicyclo[4.3.0]nona-2-ene (245 mg, 0.63 mmol) was dissolved in THF (4 ml). Then, a THF solution of disiamylborane (0.91 M 1.7 ml, 1.58 mmol) was dropwise added at 0°C. The mixture was stirred at 0°C for one hour. After an addition of a 6N sodium hydroxide aqueous solution (1.13 ml, 6.78 mmol) and a 30% hydrogen peroxide aqueous solution (0.94 ml, 8.29 mol) at 0°C, the mixture was stirred at room temperature for one hour. After an addition of water, the mixture was extracted with ethyl acetate. The organic layer was washed a sodium thiosulfate aqueous solution and a saturated sodium chloride aqueous solution, and dried over anhydrous magnesium sulfate. Then, the solvent was distilled off. The residue was purified by silica gel column chromatography (ethyl ether:n-hexane = 1:3) to obtain 3-(2-hydroxyethyl)-7-exo-t-butyldimethylsilyloxymethyl-8-endo-tetrahydropyranyloxy-cis-bicyclo[4,3,0]nona-2-ene (259 mg, 100%).

IR νmax (neat) : 3450, 2930, 2860, 1475, 1255, 1200, 835, 775 cm⁻¹.

NMR ($CDCl_3$) δ : 5.40(bs, 1H), 4.56(bs, 1H), 0.90(s, 9H), 0.50(s, 6H).

Mass (m/z) (%) : 410(M , trace), 326 (7), 269 (9), 177 (44), 159 (91), 85 (100), 41 (30).

Reference Example 3

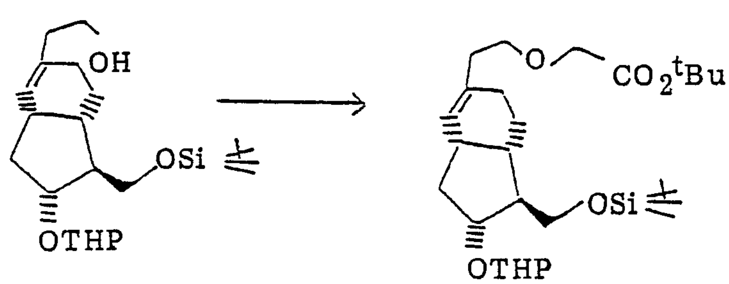

Under an argon atmosphere, 3-(2-hydroxyethyl)-7-exo-t-butyldimethylsilyloxymethyl-8-endo-tetrahydropyranyloxy-cis-bicyclo[4,3,0]nona-2-ene (295 mg, 0.718 mmol) was dissolved in methylene chloride (3 ml). Then, tertbutyl bromoacetate (3.6 ml, 22.2 mmol), a 50% sodium hydroxide aqueous solution (1.4 ml) and tetrabutylammonium hydrogensulfate (71 mg) were added thereto at room temperature, and the mixture was stirred at the same temperature for 3 days. Then, ice water (10 ml) was added, and the mixture was extracted with ethyl ether. The extract was washed with a saturated sodium chloride aqueous solution and dried over anhydrous magnesium sulfate. Then, the solvent was distilled off. The residue was purified by silica gel column chromatography (ethyl ether:n-hexane = 1:5) to obtain 3-(3-oxa-4-t-butoxycarbonylbutyl)-7-exo-tbutyldimethylsilyloxymethyl-8-endo-tetrahydropyranyloxy-cis-bicyclo[4,3,0]nona-2-ene as a colorless oily substance (346 mg, 91.8%).

IR $\nu$ max (neat) : 2950, 1750, 1460, 1390, 1370, 1300, 1255, 1215, 1130, 1080, 1030, 940, 910, 840, 780 $cm^{-1}$.

NMR ($CDCl_3$) $\delta$: 0.05(6H, s), 0.90(9H, s), 1.50(9H, s), 1.30 - 2.06(13H, m), 2.15 - 2.45(4H, m), 3.40-3.78(7H, m), 4.12(2H, s), 4.65(1H, br-s), 5.47(1H, br-s).

MASS (m/z) (%) : 85(100), 159(100), 251(15), 384(12), 440(3, M -84).

Reference Example 4

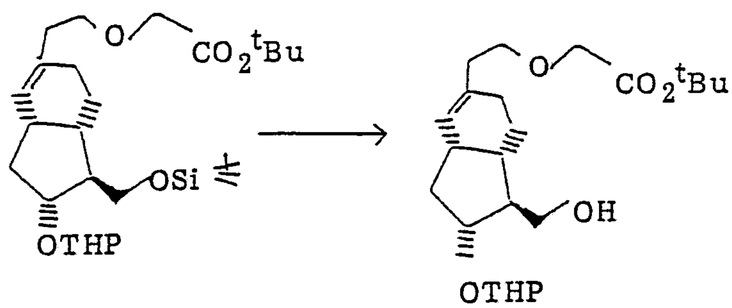

Under an argon atmosphere, 3-(3-oxa-4-t-butoxycarbonylbutyl)-7-exo-t-butyldimethylsilyloxymethyl-8-endo-tetrahydropyranyloxy-cis-bicyclo[4,3,0]nona-2-ene (345 mg, 0.657 mmol) was dissolved in THF (1 ml). Then, tetrabutylammonium fluoride (1M THF solution, 1.3 ml, 1.3 mmol) was added thereto, and the mixture was stirred at room temperature for eleven hours and 30 minutes. After an addition of a saturated sodium chloride aqueous solution, the mixture was extracted with ethyl ether, and dried over anhydrous magnesium sulfate. Then, the solvent was distilled off. The residue was purified by silica gel column chromatography (ethyl ether:n-hexane = 4:5) to obtain 3-(3-oxa-4-t-butoxycarbonylbutyl)-7-exo-hydroxymethyl-8-endo-tetrahydropyranloxy-cis-bicyclo[4,3,0]nona-2-ene (202 mg, 74.9%).

IR $\nu$max (neat) : 3500, 2950, 1750, 1440, 1370, 1300, 1230, 1200, 1130, 1080, 1020, 975, 910, 865, 845, 810 $cm^{-1}$.

NMR ($CDCl_3$) $\delta$ : 1.10 - 2.60(16H, m), 1.50(9H, s), 2.31(2H, t, J=6Hz), 3.35 - 4.20 (5H, m), 3.61(2H, t, J = 6Hz), 3.96(2H, s), 4.55 - 4.83 (1H, m), 5.45(1H, br-s).

MASS (m/z) (%) : 85 (100)

Reference Example 5

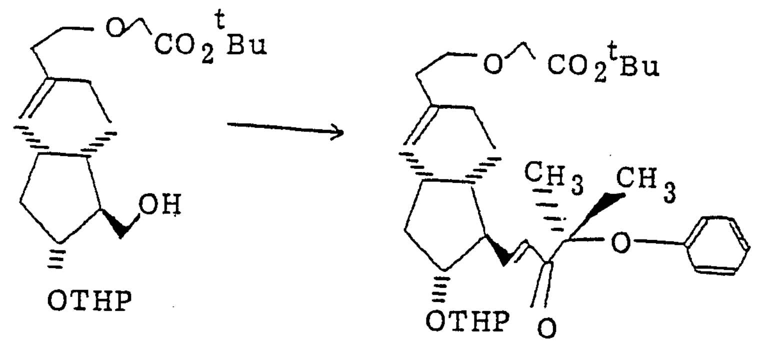

(XIIIa)

Under an argon atmosphere, 3-(3-oxa-4-t-butoxycarbonylbutyl)-7-exo-hydroxymethyl-8-endo-tetrahydropyranyloxy-cis-bicyclo[4,3,0]nona-2-ene (70 mg, 0.171 mmol) was dissolved in dimethyl sulfoxide (1.2 ml). Then, triethylamine (0.14 ml, 1.03 mmol) and a dimethyl sulfoxide solution (2 ml) of a sulfur trioxide-pyridine complex (163.6 mg, 1.03 mmol), was added thereto, and the mixture was stirred at room temperature for 30 minutes. After an addition of ice water, the mixture was extracted with ethyl acetate, washed with water and a saturated sodium chloride aqueous solution, and dried over anhydrous magnesium sulfate. Then, the solvent was distilled off to obtain 3-(3-oxa-4-t-butoxycarbonylbutyl)-7-exo-formyl-8-endotetrahydropyranyloxy-cis-bicyclo[4,3,0]nona-2-ene (71 mg, 100%).

On the other hand, sodium hydride (oily 60%, 12 mg, 0.3 mmol) was washed with pentane and suspended in THF (3 ml). Then, a THF solution (3 ml) of dimethyl[(2-oxo-3-methyl-3-phenoxy)butyl]-phosphonate (128.7 mg, 0.45 mmol) was added thereto, and the mixture was stirred at room temperature for 40 minutes. Then, a THF solution (3 ml) of 3-(3-oxa-4-t-butoxycarbonylbutyl)-7-exo-formyl-8-endotetrahydropyranyloxy-cis-bicyclo[4,3,0]nona-2-ene (71 mg) was added thereto, and the mixture was stirred at 50°C for five hours. After an addition of a saturated ammonium chloride aqueous solution, the mixture was extracted with ethyl ether. The ethyl ether layer was washed with a saturated sodium chloride aqueous solution, and dried over anhydrous magnesium sulfate. Then, the solvent was distilled off, and the residue thus obtained was purified by silica gel column chromatography (ethyl ether:n-hexane = 1:3) to obtain 3-(3-oxo-4-t-butoxycarbonylbutyl)-7-exo-(3-oxo-4-methyl-4-phenoxy-trans-1-pentenyl)-8-endo-tetrahydropyranyloxy-cis-bicyclo[4,3,0]nona-2-ene (XIIIa) (62 mg, 84%).

IR ν max (neat) : 2940, 1750, 1670, 1620, 1130, 750 695 $cm^{-1}$.
NMR ($CDCl_3$) δ : 6.56 - 7.31(7H, m), 5.34(1H, bs), 4.65(1H, bs), 3.94(2H, s), 1.47(15H, s).

Reference Examples 6 to 10

In the same manner as in Reference Example 4, XIIIc - XIIIg were prepared from 3-(3-oxa-4-t-butoxycarbonyl)-7-exo-hydroxymethyl-8-endo-tetrahydropyranyloxy-cis-bicyclo[4,3,0]nona-2-ene. The spectrum data of compounds XIIIc - XIIIg are shown in Table I.

Table 1

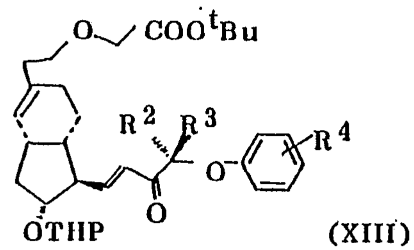

(XIII)

| Reference Example | Compound | | | | IR Spectra | NMR Spectra |
|---|---|---|---|---|---|---|
| | No. | $R^2$ | $R^3$ | $R^4$ | $\nu_{max}$ (neat) ; $cm^{-1}$ | δ ($CDCl_3$) |
| 6 | XIIIc | H | H | H | 2950, 1745, 1700, 1620, 1130, 730, 690 | 6.79-7.42(6H,m), 6.48(1H,dd,J=15Hz,3Hz), 5.36(1H,bs), 4.67(2H,s), 3.90(2H,s), 1.50(9H,s) |
| 7 | XIIId | H or $CH_3$ | $CH_3$ or H | H | 2940, 2860, 1745, 1690, 1620, 1135, 750, 690 | 6.72-7.40(6H,m), 6.53(1H,dd,J=15Hz,3Hz), 5.37(1H,bs), 4.25-4.50(1H,m), 3.95(2H,s), 3.59(2H,t,J=6Hz), 1.49(9H,m) |
| 8 | XIIIe | H | H | p-$OCH_3$ | 2940, 2860, 1745, 1685, 1620, 1135, | 6.90-7.20(1H,m), 6.84(4H,s), 6.49(1H,dd, J=15Hz,3Hz), 5.38(1H,bs), 4.66(2H,s), 4.40-4.60(1H,m), 3.96(2H,s), 3.77(3H,s), 3.60(2H,t,J=6Hz), 1.48(9H,s) |
| 9 | XIIIf | H | H | p-F | 2940, 2860, 1745, 1715, 1685, 1620, 1135 | 6.67-7.32(5H,m), 6.56(1H,dd,J=15Hz,3Hz), 5.38(1H,bs), 4.67(2H,s), 4.40-4.60(1H,m), 3.95(2H,s), 1.47(9H,s) |
| 10 | XIIIg | H | H | m-F | 2930, 2870, 1745, 1715, 1690, 1615, 1130 | 6.77-7.37(5H,m), 6.52(1H,dd,J=15Hz,3Hz), 5.42(1H,bs), 4.78(2H,s), 4.58-4.72(1H,m), 3.97(2H,s), 1.50(9H,s) |

Synthesis Example 1

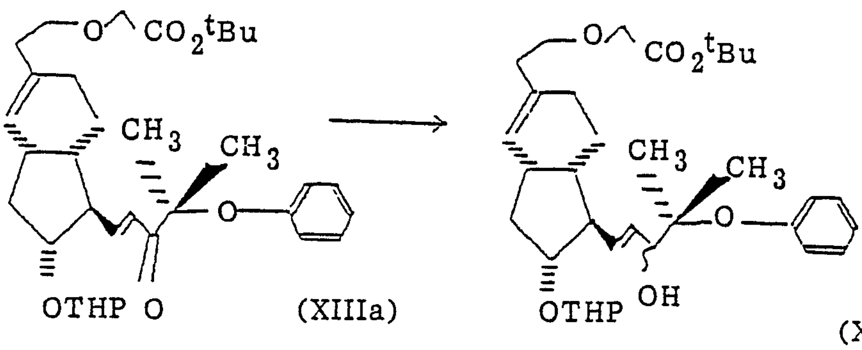

(XIIIa) (XIVa)

Under an argon atmosphere, 3-(3-oxa-4-t-butoxycarbonylbutyl)-7-exo-(3-oxo-4-methyl-4-phenoxy-trans-l-pentenyl)-8-endo-tetrahydropyranyloxy-cis-bicyclo[4,3,0]nona-2-ene (XIIIa) (88 mg, 0.l5 mmol) was dissolved in methanol (3 ml). Then, the solution was cooled to -l5°C and an excess amount of sodium borohydride was added thereto. The mixture was stirred at -l5°C for 30 minutes, and an excess amount of acetone was added thereto. The mixture was returned to room temperature, and a saturated ammonium chloride aqueous solution was added thereto. The mixture was extracted with ethyl acetate, and the extract was dried over anhydrous magnesium sulfate. Then, the solvent was distilled off to obtain 3-(3-oxa-4-t-butoxycarbonylbutyl)-7-exo-(3-hydroxy-4-methyl-4-phenoxy-trans-l-pentenyl)-8-endo-tetrahydropyranyloxy-cis-bicyclo[4,3,0]nona-2-ene (XIVa) (95 mg, l00%).

IR $\nu$max (neat) : 3490, 2940, l745, ll30, 780, 700 cm$^{-1}$.

NMR ($CDCl_3$) $\delta$ : 6.65 - 7.33(5H, m), 5.56 - 5.79(2H, m), 5.45(lH, bs), 4.50 - 4.75(lH, m), 3.95(2H, s), 3.58-(2H, t, J = 6$H_z$), l50(9H, s), l.25(6H, 3).

Synthesis Examples 2 to 6

In the same manner as in Synthesis Example l, XlVc-XlVg were prepared from, XIIIc - XIIIg, respectively. The spectrum data of compounds XIVc - XIVg are shown in Table 2.

Table 2

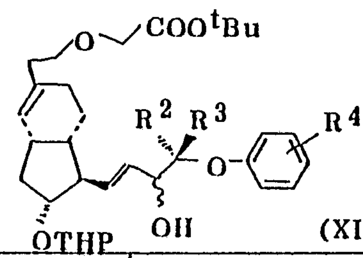
(XIV)

| Synthesis Example | Compound | | | | IR Spectra | NMR Spectra |
|---|---|---|---|---|---|---|
| | No. | $R^2$ | $R^3$ | $R^4$ | $\nu_{max}$ (neat) : $cm^{-1}$ | δ ($CDCl_3$) |
| 2 | XIVc | H | H | H | 3450, 2950, 1745, 1130, 730, 690 | 6.80-7.49(5H,m), 5.60-5.82(2H,m), 5.47 (1H,bs), 4.50-4.75(1H,m), 3.92(2H,s), 3.58(2H,t,J=6Hz), 1.50(9H,s) |
| 3 | XIVd | H or $CH_3$ | $CH_3$ or H | H | 3450, 2940, 2860, 1745, 1135, 750, 690 | 6.80-7.43(5H,m), 5.59-5.83(2H,m), 5.40 (1H,bs), 4.66(1H,bs), 3.97(2H,s), 3.60 (2H,t,J=6Hz), 1.48(9H,s) |
| 4 | XIVe | H | H | p-$OCH_3$ | 3450, 2940, 2860, 1740, 1135 | 6.85(4H,s), 5.60-5.85(2H,m), 5.39(1H,bs), 4.66(1H,bs), 3.97(2H,s), 3.79(3H,s), 3.63 (2H,t,J=6Hz), 1.48(9H,s) |
| 5 | XIVf | H | H | p-F | 3450, 2940, 2860, 1745, 1135 | 6.72-7.13(4H,m), 5.61-5.86(2H,m), 5.40 (1H,bs), 4.66(1H,bs), 3.96(2H,s), 1.48 (9H,s) |
| 6 | XIVg | H | H | m-F | 3450, 2920, 2860, 1740, 1135 | 6.78-7.10(4H,m), 5.60-5.87(2H,m), 5.40 (1H,bs), 4.67(1H,bs), 3.97(2H,s), 1.50 (9H,s) |

Synthesis Example 7

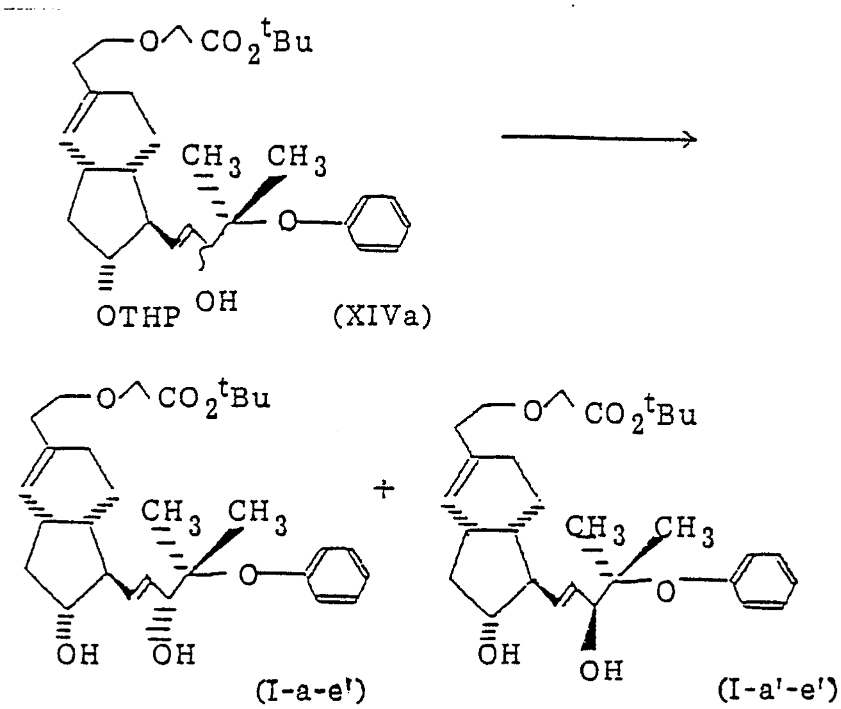

(XIVa)

(I-a-e')

(I-a'-e')

3-(3-oxa-4-t-butylcarbonylbutyl)-7-exo-(3-hydroxy-4-methyl-4-phenoxy-trans-l-pentenyl)-8-endotetrahydropyranyloxy-cis-bicyclo[4,3,0]nona-2-ene (XIVa) (95 mg, 0.l5 mmol) was dissolved in a mixture of acetic acid:THF:water (5 ml) (volume ratio of 3:l:l), and the solution was stirred at 50°C for l4 hours. After cooling, the solution was diluted with ethyl acetate and neutralized with a saturated sodium bicarbonate aqueous solution. The ethyl acetate layer was washed with a saturated sodium chloride aqueous solution, and dried over anhydrous magnesium sulfate. The residue obtained by distilling off the solvent, was purified by silica gel column chromatography (ethyl ether:n-hexane = l:l) to obtain 3-(3-oxa-4-t-butoxycarbonylbutyl)-7-exo-(3α-hydroxy-4-methyl-4-phenoxyl-trans-l-pentenyl)-8-endo-hydroxy-cis-bicyclo-[4,3,0]nona-2-ene (l-a-e') (35 mg, 46.5%) as a high polarity fraction and 3-(3-oxa-4-t-butoxycarbonylbutyl)-7-exo-(3β-hydroxy-4-methyl-4-phenoxy-trans-l-pentenyl)-8-endo-hydroxy-cis-bicyclo[4,3,0]nona-2-ene (l-a'-e') (29 mg, 38.5%) as a low polarity fraction, respectively.

The spectrum data of the α-epimer are as shown below. The spectrum of the β-epimer was substantially the same.

IR νmax (neat) : 3400, 2930, l745, ll30, 780, 700 $cm^{-1}$.

NMR ($CDCl_3$) δ : 6.72 - 7.3l(5H, m), 5.50 - 5.80(2H, m), 5.40(lH, bs), 3.95(2H, s), 3.60 (2H, t, J = 6$H_z$), l.47(9H, s), l.23(6H, s).

Synthesis Examples 8 to l2

In the same manner as in Synthesis Example 7, l-c-e' -l-g-e' were prepared from XIVc - XIVg, respectively. The spectrum data of compounds l-c-e' -l-g-e' are shown in Table 3.

Table 3

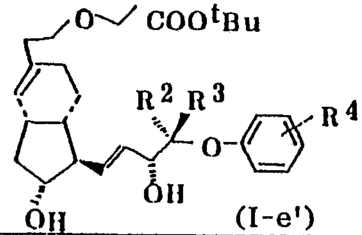

(I-e')

| Synthesis Example | Compound | | | | IR Spectra | NMR Spectra |
|---|---|---|---|---|---|---|
| | No. | $R^2$ | $R^3$ | $R^4$ | $\nu_{max}$ (neat) : $cm^{-1}$ | $\delta$ ($CDCl_3$) |
| 8 | I-c-e' | H | H | H | 3400, 2940, 1745, 1130, 750, 690 | 6.83-7.42(5H,m), 5.60-5.81(2H,m), 5.42 (1H,bs), 3.95(2H,s), 3.60(2H,t,J=6Hz), 1.47(9H,s) |
| 9 | I-d-e' | H or $CH_3$ | $CH_3$ or H | H | 3400, 2920, 2860, 1745, 1135, 750, 690 | 6.80-7.42(5H,m), 5.57-5.76(2H,m), 5.40 (1H,bs), 3.96(2H,s), 3.60(2H,t,J=6Hz), 1.50(9H,s) |
| 10 | I-e-e' | H | H | p-$OCH_3$ | 3400, 2930, 2860, 1740, 1135 | 6.86(4H,s), 5.60-5.82(2H,m), 5.42(1H, bs), 3.96(2H,s), 3.80(3H,s), 3.61(2H, t,J=6Hz), 1.50(9H,s) |
| 11 | I-f-e' | H | H | p-F | 3400, 2920, 2860, 1740, 1135 | 6.73-7.12(4H,m), 5.60-5.82(2H,m), 5.42 (1H,bs), 3.95(2H,s), 3.60(2H,t,J=6Hz), 1.50(9H,s) |
| 12 | I-g-e' | H | H | m-F | 3400, 2920, 2860, 1745, 1135 | 6.50-7.30(4H,m), 5.55-5.80(2H,m), 5.42 (1H,bs), 3.95(2H,s), 3.60(2H,t,J=6Hz), 1.48(9H,s) |

Synthesis Example 13

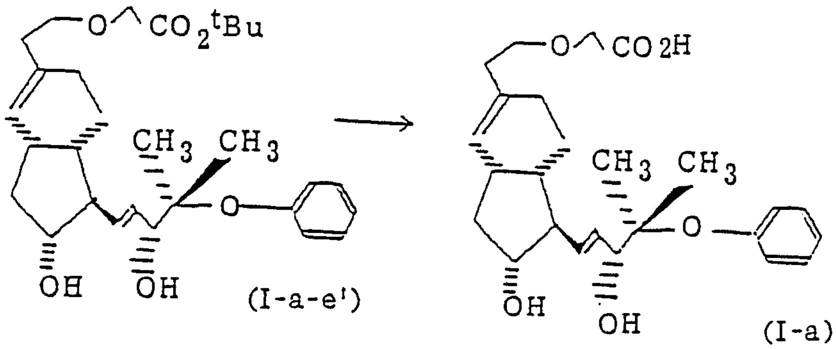

(I-a-e') (I-a)

Under an argon atmosphere, 3-(3-oxa-4-t-butoxycarbonylbutyl)-7-exo-(3α-hydroxy-4-methyl-4-phenoxy-trans-l-pentenyl)-8-endo-hydroxy-cis-bicyclo-[4,3,0]nona-2-ene (I-a-e') (35 mg, 0.072 mmol) was dissolved in methanol (0.8 ml). Then, a 7% potassium hydroxide aqueous solution (0.8 ml) was added thereto at 0°C, and the mixture was stirred for overnight. After neutralizing the mixture with a IN hydrochloric acid aqueous solution at 0°C, the methanol layer was washed with a n-pentane and the residual aqueous layer was adjusted to pH4 with a IN hydrochloric acid aqueous solution. Then, it was extracted with ethyl acetate, washed with a saturated sodium chloride aqueous solution and dried over anhydrous magnesium sulfate. Then, the solvent was distilled off to obtain 3-(3-oxa-4-carboxybutyl)-7-exo-(3α-hydroxy-4-methyl-4-phenoxyl-trans-l-pentenyl)-8-endo-hydroxy-cis-bicyclo-[4,3,0]nona-2-ene (I-a) (24.4 mg, 78.7%).

IR νmax (neat) : 3400, 2920, I730, II30, 780, 700 $cm^{-1}$.

NMR ($CDCl_3$) δ : 6.60 - 7.43(5H, m), 5.50 - 5.76(2H, m), 5.43(IH, bs), 4.07(2H, s), 3.46(2H, t, J = $6H_z$), I.23(6H, s).

Synthesis Examples I4 to I8

In the same manner as in Synthesis Example I3, I-c - I-g were prepared from I-c-e' -I-g-e', respectively. The spectrum data of compounds I-c - I-g are shown in Table 4.

Table 4

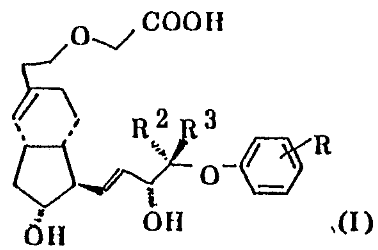

(I)

| Synthesis Example | Compound | | | | IR Spectra | NMR Spectra |
|---|---|---|---|---|---|---|
| | No. | $R^2$ | $R^3$ | $R^4$ | $\nu_{max}$ (neat) : $cm^{-1}$ | δ ($CDCl_3$) |
| 14 | I-c | H | H | H | 3400, 2940, 1740, 1140, 750, 690 | 6.70-7.45(5H,m), 5.55-5.80(2H,m), 5.43 (1H,bs), 4.07(2H,s), 3.46(2H,t,J=6Hz) |
| 15 | I-d | H or $CH_3$ | $CH_3$ or H | H | 3400, 2920, 2860, 1735, 1140, 750, 690 | 6.80-7.43(5H,m), 5.50-5.76(2H,m), 5.40 (1H,bs), 5.30-5.70(3H,m), 4.05(2H,s) |
| 16 | I-e | H | H | p-$OCH_3$ | 3400, 2920, 2850, 1730, 1140 | 6.85(4H,s), 5.60-5.77(2H,m), 5.41(1H, bs), 4.09(2H,s), 3.78(3H,s) |
| 17 | I-f | H | H | p-F | 3400, 2920, 1730, 1120 | 6.72-7.15(4H,m), 5.58-5.80(2H,m), 5.42 (1H,bs), 5.30-5.70(3H,m), 4.08(2H,s) |
| 18 | I-g | H | H | m-F | 3400, 2930, 1740, 1140 | 6.40-7.40(4H,m), 5.58-5.82(2H,m), 5.42 (1H,bs), 5.30-5.70(3H,m), 4.07(2H,s) |

Synthesis Example I9

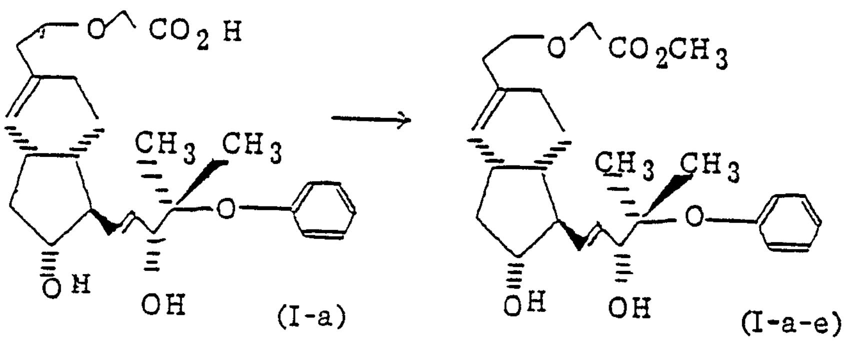

(I-a) (I-a-e)

3-(3-Oxa-4-carboxybutyl)-7-exo-(3α-hydroxy-4-methyl-4-phenoxy-trans-l-pentenyl)-8-endo-hydroxy-cis-bicyclo[4.3,0]nona-2-ene (I-a) (20 mg, 0.0464 mmol) was dissolved in methanol (2 ml) and an ethereal diazomethane was added until yellow color persisted. The mixture was quenched by the addition of 0.l ml of formic acid.

Then, the solvent was distilled off. The residue was purified by silica gel column chromatography (ethyl ether:n-hexane = I0:I) to obtain 3-(3-oxa-4-methoxycarbonylbutyl)-7-exo-(3α-hydroxy-4-methyl-4-phenoxy-trans-l-pentenyl)-8-endo-hydroxy-cis-bicyclo[4,3,0]nona-2-ene (I-a-e) (I8 mg, 87.I%).

IR νmax (neat) : 3400, 2920, 2870, I755, II35, 780, 700 $cm^{-1}$

NMR ($CDCl_3$) δ : 6.86 - 7.42(5H, m), 5.58 - 5.79(2H, m), 5.42(IH, bs), 4.09(2H, S), 3.75(3H, S) 3.62(2H, t, J = 6Hz), I.24(6H, S)

Test Example I. Ethanol ulcer inhibiting activity

Male wistar rats having a weight of from I70 to 270 g were put into individual cages and starved for 24 hours. During this starvation period, the rats were allowed to drink water freely. A test compound or $PGE_2$ as a comparative compound was orally administered, and 0.5 or 4.0 hour later, I ml of 99.5% ethanol was orally administered to each rat. One hour after the ethanol administration, each rat was filled by cervical vertebral luxation, and the stomach was taken out. A I% formaline solution was injected into the stomach in an amount of about 8 ml, and the stomach was fixed in the formaline solution for 30 minutes. After the fixing, the stomach was severed along the greater curvature, and the mucous membrane surface of the stomach was gently washed with flowing water. Then, the total length of damages appearing on the gastric glandular portion was obtained and used as the ulcer index.

The ethanol ulcer inhibiting activity of a test compound was represented by $ED_{50}$ (i.e. the dose of the test compound at which the ulcer was inhibited by 50% relative to the ulcer index of the non-treated group). The results are shown in column (A) in Table 5. To the non-treated group, the solvent was administered.

Test Example 2. Hydrochloric acid ulcer inhibiting activity

Male wistar rats having a weight of from I90 to 240 g were used. The rats were starved in the same manner as in Test Example 6, and the test compounds and $PGE_2$ were, respectively, orally administered in an amount of 30 μg/kg, and 30 minutes later, I ml of 0.6N hydrochloric acid was orally administered. Thirty minutes after the hydrochloric acid administration, each rat was killed by cervical vertebral luxation. Then, the stomach was taken out and fixed, and the ulcer index was obtained, in the same manner as in Test

Example 6. The hydrochloric acid ulcer inhibiting activity of a test compound was represented by the inhibiting percentage (i.e. the percentage of the ulcer index of the treated group to the ulcer index of the non-treated group). The results are shown in the column (B) in Table 5. To the non-treated group, the solvent was administered.

Test Example 3. Indomethacin ulcer inhibiting activity

Male wistar rats having a weight of from 2l0 to 270 g were used. The rats were starved in the same manner as in Test Example 6. Then, 60 µg/kg of a test compound or 30 µg/kg of $PGE_2$ was orally administered to each rat, and 30 minutes later, 60 mg/kg of indomethacin was hypodermically administered. Three hours after the indomethacin administration, the test compound or $PGE_2$ was again orally administered in the same amount. Six hours after the first administration of indomethacin, each rat was killed by cervical bertebral luxation. The stomach was taken out and fixed, and the ulcer index was obtained, in the same manner as in Test Example 6.

The indomethacin ulcer inhibiting activity of a test compound was represented by the inhibiting percentage (i.e. the inhibiting percentage of the ulcer index of the treated group to the ulcer index of the non-treated group). The results are shown in the column (C) in Table 5. To the non-treated group, the solvent was administered.

Now, Formulation Examples for the formulation of compounds used in the preceding Test Examples, will be given.

Formulation Example I

Compound: 500 mg
Patato starch: l50 mg
Silicic anhydride: 50 mg
Magnesium stearate: l0 mg
Lactose: Balance to the total of l,000 mg

The above ingredients were uniformly mixed and filled into hard capsules in an amount of 200 mg each.

Formulation Example 2

Compound: 500 mg
Potato starch: l00 mg
Crystalline cellulose: 60 mg
Silicic anhydride: 50 mg
Hydroxypropyl cellulose: 30 mg
Magnesium stearate: l5 mg
Lactose: Balance to the total of 00 mg

Now, Formulation Examples for the formulation of compounds used in the preceding Test Examples, will be given.

Formulation Example I

Compound: 500 mg
Patato starch: l50 mg
Silicic anhydride: 50 mg
Magnesium stearate: l0 mg
Lactose: Balance to the total of l,000 mg

The above ingredients were uniformly mixed and filled into hard capsules in an amount of 200 mg each.

Formulation Example 2

Compound: 500 mg
Potato starch: 100 mg
Crystalline cellulose: 60 mg
Silicic anhydride: 50 mg
Hydroxypropyl cellulose: 30 mg
Magnesium stearate: 5 mg
Lactose: Balance to the total of 1,000 mg

The active ingredients, lactose, potato starch, crystalline cellulose and silicic anhydride were mixed. After an addition of a methanol solution containing 10% of hydroxypropyl cellulose was added thereto, and the mixture was kneaded and granulated. Then, it was extruded through a screen having openings with a diameter of 0.8 mm, to obtain granules. After drying the granules, the magnesium stearate was added thereto, followed by compression molding to obtain tablets each having a weight of 200 mg

Formulation Example 3

Compound: 500 mg
Propylene glycol: Balance to the total of 10 ml

The active ingredient was dissolved in propylene glycol, and the solution was asceptically filtered and then filled into ampules in an amount of 0.2 ml each.

Formulation Example 4

Compound: 250 mg
Polyethylene glycol 1,500: 3,000 mg
Polyethylene glycol 6,000: Balance to the total of 5,000 mg

The above ingredients were heat-melted at 60°C and uniformly mixed, and the mixture was poured into a plastic mold and cooled to obtain a suppository of 1 g.

Table 5

| Compound (Compound No.) | (A) Ethanol ulcer inhibiting activity $LD_{50}$% ( µg/Kg, P.O.) ( Activity ratio ) | | (B) Hydrochloric acid ulcer inhibiting activity inhibiting rate (%) | (C) Indomethacin ulcer inhibiting activity Inhibiting rate (%) |
|---|---|---|---|---|
| | 1.5 hr | 5.0 hr | | |
| $PGE_2$ 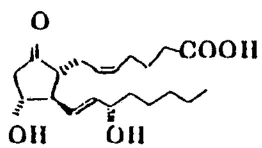 | 9.0 (1.0) | 362 | 61.6 % inhibition by oral administration of 30 µg/Kg | 98.3 % inhibition by oral administration of 30 µg/Kg twice |
| Prostacyclin 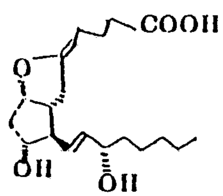 | No inhibiting activity observed by oral administration of 30 µg/kg | | No inhibiting activity observed by oral administration of 30 µg/kg | No inhibiting activity observed by oral administration of 60 µg/kg twice. |
| (I-a) 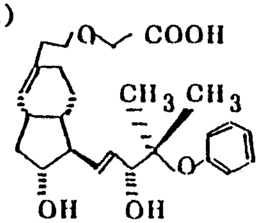 | 1.1 (8.21) | 25 | 63 % inhibition by oral administration of 30 µg/Kg | 45.3 % inhibition by oral administration of 30 µg/Kg |

Table 5 (cont'd)

| Compound (Compound No.) | (A) Ethanol ulcer inhibiting activity $LD_{50}$% ( μg/Kg, P.O.) ( Activity ratio ) | | (B) Hydrochloric acid ulcer inhibiting activity inhibiting rate (%) | (C) Indomethacin ulcer inhibiting activity Inhibiting rate (%) |
|---|---|---|---|---|
| | 1.5 hr | 5.0 hr | | |
| (I-a-e) 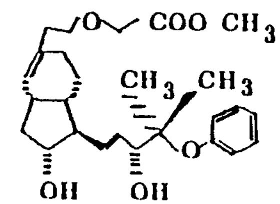 | 3.9 (2.32) | 9.4 | 66 % inhibition by oral administration of 30 μg/Kg | 73 % inhibition by oral administration of 30μg/Kg |
| (I-c) 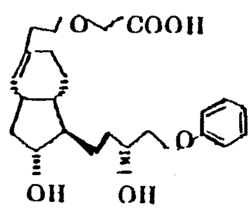 | 8.9 (1.01) | 98 | 19.2% inhibition by oral administration of 30 μg/Kg<br>62.1 % inhibition by oral administration of100μg/Kg | 14.6% inhibition by oral administration of30 μg/Kg<br>54.9 % inhibition by oral administration of100μg/Kg |
| (I-d) 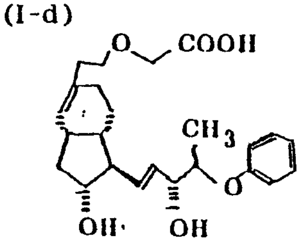 | 4.7 (1.92) | 86 | 19.5 % inhibition by oral administration of 30 μg/Kg<br>62.1 % inhibition by oral administration of100μg/Kg | 14.6 % inhibition by oral administration of 30 μg/Kg<br>54.9 % inhibition by oral administration of100μg/Kg |

Table 5 (cont'd)

| Compound (Compound No.) | (A) Ethanol ulcer inhibiting activity $ED_{50}$※ ( μg/Kg. P.O.) ( Activity ratio ) | | (B) Hydrochloric acid ulcer inhibiting activity inhibiting rate (%) | (C) Indomethacin ulcer inhibiting activity Inhibiting rate (%) |
|---|---|---|---|---|
| | 1.5 hr | 5.0 hr | | |
| (I-e) 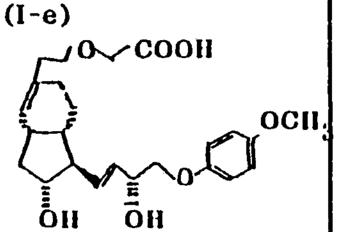 | 19.4 (1/2.15) | 130 | | |
| (I-f) 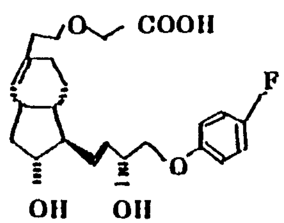 | 8.2 (1.10) | 83 | | |
| (I-g) 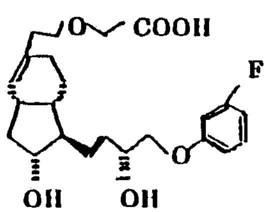 | 16.8 (1/1.86) | 92 | | |

## Claims

1. A prostacyclin analogue having the formula:

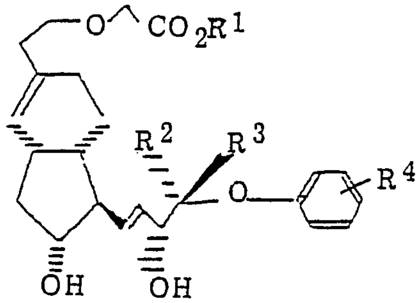

(I)

wherein $R^1$ is a hydrogen atom or a lower alkyl group: $R^2$ and $R^3$ each is a hydrogen atom or a lower alkyl group: $R^4$ is a hydrogen atom, a halogen atom or a lower alkoxy group.

2. An anti-ulcer composition comprising an effective amount of the prostacyclin analogue as defined in Claim I and a pharmaceutically acceptable carrier.